# EUROPEAN PATENT APPLICATION

(11) **EP 4 681 609 A1**
(43) Date of publication of application: **21.01.2026**
(21) Application number: 25185753.8
(22) Date of filing: 27.06.2025
(51) Int. Cl.: A61B 5/00, A61B 5/145, A61B 5/1455

(54) **SYSTEM FOR ILLUMINATING ANALYTE AND SYSTEM FOR TESTING ANALYTE**

(30) Priority: 16.07.2024 CN 202410951464
(71) Applicant: SenSura Pte. Ltd, 608526 Singapore (SG)
(72) Inventor: ZHENG, Hongzhi, Guangzhou, 510663 (CN)
(74) Representative: Metida

(57) **Abstract**

The present invention provides a system for illuminating an analyte and a system for testing an analyte which relate to the field of optical analysis. The system for illuminating the analyte includes a shell and a light source. The light source is capable of providing light within a preset wavelength range; and the shell is capable of fitting a tested part of a tested object and forming an imaging area at a fitting position, and the light source illuminates the imaging area. According to this application, a wavelength range of light provided by the light source covers both a wavelength range within which analyte distribution data can be obtained and a wavelength range within which analyte spectral data can be obtained, and the light source is set as a ring-shaped light source, to provide stable and uniform illumination for the imaging area.

## Description

### TECHNICAL FIELD

The present invention relates to the field of optical analysis, and in particular to a system for illuminating an analyte and a system for testing an analyte.

### BACKGROUND

Non-invasive measurement usually means non-intrusive measurement and is in contrast to invasive measurement, which means that a measurement instrument is usually placed in contact with the skin of a measured object to indirectly guide or sense physiological and biochemical parameters of a living organism, and is also known as indirect measurement. Currently, commonly used non-invasive measurement technologies include an electrochemical method and an optical method.

Glucose testing in a human body is used as an example. An existing patent document with a publication No. CN118078277A discloses a non-invasive blood glucose testing method based on hyperspectral data analysis. In this method, a light source is used to provided ambient light for a hyperspectral imager. In a darkroom environment, after the light source illuminates a blood vessel area on the back of a hand, the hyperspectral imager captures human skin and blood vessel areas of a tested object for a plurality of times, to obtain hyperspectral images under different concentrations. The plurality of obtained hyperspectral images are then sent to a testing device, which aggregates the images to obtain a hyperspectral image dataset including two-dimensional spatial data and one-dimensional spectral data. A test result is highly dependent on the environment, and light interference in the environment can affect the accuracy of testing, making it difficult to apply in a miniaturization scenario.

In addition, patent document US6424849B1 discloses a method for separately determining a blood glucose level using IR beams reflected from a skin surface. The apparatus is provided with some accommodating parts for holding a body part against an ATR plate, so that a selected skin surface is in contact with the ATR plate during measurement. The human skin surface is irradiated with the IR beams, and reference and measurement wavelength components in the reflected IR beams are detected and quantified. During measurement, ambient light can enter the accommodating parts through openings in the accommodating parts, so that a measurement result is susceptible to light interference from the environment, which affects the accuracy of testing.

### SUMMARY

To overcome defects in the prior art, the present invention is intended to provide a system for illuminating an analyte and a system for testing an analyte.

The system for illuminating an analyte provided according to the present invention includes a shell and a light source. The light source is capable of providing light within a preset wavelength range; and the shell is capable of fitting a tested part of a tested object and forming an imaging area at a fitting position, and the light source illuminates the imaging area.

Further, the light source includes a single light source, and a wavelength range of light provided by the single light source covers both a wavelength range within which analyte distribution data can be obtained and a wavelength range within which analyte spectral data can be obtained.

Further, the light source includes a plurality of light-emitting modules, which are evenly distributed on a same circumference.

Further, the light source includes two light sources, a wavelength range of light provided by one light source covers a wavelength range within which analyte distribution data can be obtained, and a wavelength range of light provided by the other light source covers a wavelength range within which analyte spectral data can be obtained.

Further, the two light sources respectively include a plurality of light-emitting modules, the plurality of light-emitting modules of the two light sources are arranged alternately on a same circumference, and the light-emitting modules are evenly distributed on the same circumference.

Further, one light source provides infrared light with a wavelength range of 800 nm to 1,000 nm; and the other light source provides ultraviolet light with a wavelength range of 300 nm to 390 nm.

Further, a first bandpass filter is also disposed in the shell, and the first bandpass filter is located between the light source and the imaging area; and the first bandpass filter allows the light within the preset wavelength range to pass through and light outside the preset wavelength range to be cut off.

Further, the shape and material of the shell allow the shell to fit the skin around a perimeter of the tested part of the tested object.

The system for testing an analyte according to the present invention includes a system for illuminating an analyte.

Further, the system for testing an analyte includes a portable apparatus, wherein a system for illuminating an analyte is integrated in the portable apparatus.

Compared with the conventional technologies, the present invention has the following beneficial effects.
1. According to this application, a wavelength range of light provided by the light source covers both a wavelength range within which analyte distribution data can be obtained and a wavelength range within which analyte spectral data can be obtained, and the light source is set as a ring-shaped light source, to provide stable and uniform illumination for the imaging area. During testing, electrochemical reaction with the analyte is not required, and a testing manner is more easily implemented, especially when an analyte in a living body is tested, so that non-invasive testing can be achieved.
2. In this application, spectral data of different areas can be obtained by utilizing uneven distribution of the analyte in the imaging area. Because other components in the imaging area except the analyte are distributed relatively uniformly, a difference in spectral data in different areas can directly demonstrate the information about the analyte (such as a concentration of the analyte) correlated to the spectral data after the influence of a non-analyte is excluded.
3. In this application, fluorescence spectroscopy is used for testing, preventing a traditional Raman method for measuring the analyte, thereby achieving low costs and miniaturization of the testing system and achieving real-time testing.

### BRIEF DESCRIPTION OF DRAWINGS

Other features, objects, and advantages of the present invention will become more apparent by reading detailed description of non-limiting embodiments with reference to the following drawings:
FIG. 1 is a schematic structural diagram of a device for testing an analyte provided in a first embodiment;
FIG. 2 is a schematic diagram of a watch structure provided in the first embodiment;
FIG. 3 is a schematic exploded diagram of a watch internal structure provided in the first embodiment;
FIG. 4 is a schematic diagram of a watch wearing structure provided in the first embodiment;
FIG. 5 is a schematic diagram of a watch back structure provided in the first embodiment;
FIG. 6 is a flow chart in a second embodiment;
FIG. 7 is a schematic diagram of a first image collected in a third embodiment;
FIG. 8 is a schematic diagram of a second image collected in the third embodiment;
FIG. 9 is a schematic diagram of a testing model in the third embodiment;
FIG. 10 is a schematic diagram of testing point-reference point spectral data obtained in the third embodiment;
FIG. 11 is an experimental result of analysis result accuracy of an analysis model; and
FIG. 12 is a schematic structural diagram of an electronic device provided in a sixth embodiment;

In the accompanying drawings,
100: imaging area; 200: testing device;
201: light source; 202: imaging spectrum detection apparatus;
203: controller; 204: first bandpass filter;
205: lens; 206: second bandpass filter;
207: circuit board; 501: processor;
502: memory.

### DETAILED DESCRIPTION OF EMBODIMENTS

The present invention is described below in detail with reference to specific embodiments. The following embodiments help those skilled in the art further understand the present invention, but do not limit the present invention in any form. It should be noted that those skilled in the art may make some improvements and transformation without departing from the idea of the present invention. The improvements and transformation shall fall within the protection scope of the present invention.

### First embodiment

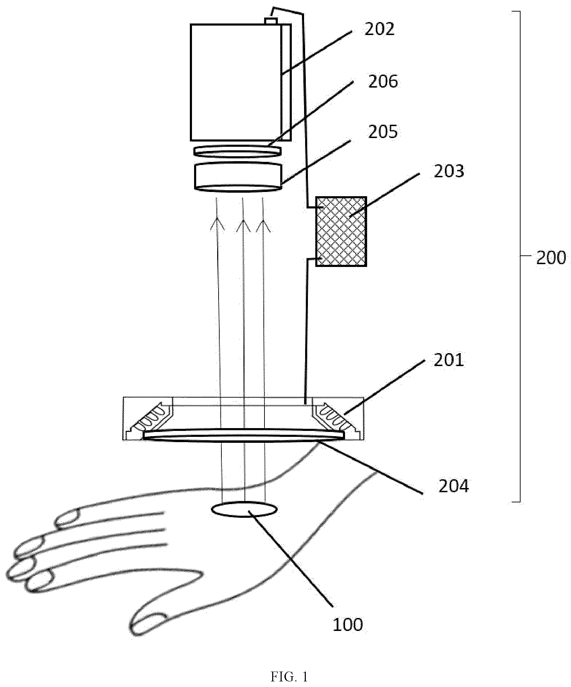

FIG. 1 is a structural diagram of this embodiment. This embodiment provides a system for illuminating an analyte, including a shell and a light source 201. The light source 201 can provide light within a preset wavelength range. The shell is capable of fitting a tested part of a tested object and forming an imaging area 100 at a fitting position, and the light source 201 illuminates the imaging area 100.

The light source 201 includes a single light source 201, and a wavelength range of light provided by the single light source 201 covers both a wavelength range within which analyte distribution data can be obtained and a wavelength range within which analyte spectral data can be obtained. The light source 201 includes a plurality of light-emitting modules, which are evenly distributed on a same circumference.

The light source 201 includes two light sources 201. A wavelength range of light provided by one light source 201 covers a wavelength range within which analyte distribution data can be obtained, and a wavelength range of light provided by the other light source 201 covers a wavelength range within which analyte spectral data can be obtained. The two light sources 201 respectively include a plurality of light-emitting modules, the plurality of light-emitting modules of the two light sources 201 are arranged alternately on a same circumference, and the light-emitting modules are evenly distributed on the same circumference. One light source 201 provides infrared light with a wavelength range from 800 nm to 1,000 nm. The other light source 201 provides ultraviolet light with a wavelength range of 300 nm to 390 nm.

The shape and material of the shell allow the shell to fit the skin around a perimeter of the tested part of the tested object. A first bandpass filter 204 is also disposed in the shell, and the first bandpass filter 204 is located between the light source 201 and the imaging area 100. The first bandpass filter 204 allows the light within the preset wavelength range to pass through and light outside the preset wavelength range to be cut off.

Specifically, the light source 201 is a part of a testing device 200, and the testing device 200 further includes an imaging spectrum detection apparatus, a controller 203, the first bandpass filter 204, a second bandpass filter 206, and a lens 205. The controller 203 establishes an electric connection or communication connection with the light source 201 and the imaging spectrum detection apparatus separately.

The light source 201 can provide light within a preset wavelength range. Because obtaining the distribution data and spectral data of the analyte requires irradiation by light of different wavelength ranges, there are two implementation manners: a light source 201 that can provide light with a larger wavelength range or two light sources 201 that can respectively provide light with smaller wavelength ranges. When there is one light source 201, the wavelength range of the light provided by the light source 201 needs to cover both the wavelength range within which analyte distribution data can be obtained and the wavelength range within which analyte spectral data can be obtained, such as a halogen lamp. When there are two light sources 201, the two light sources 201 provide different pieces of light. A wavelength range of the analyte distribution data can be obtained through a wavelength coverage of one piece of light, and a wavelength range of the analyte spectral data can be obtained through a wavelength coverage of the other piece of light, such as an infrared lamp combined with an ultraviolet lamp, a visible light lamp combined with an ultraviolet lamp.

To enable the imaging area 100 to be irradiated evenly, a ring-shaped light source 201 may be used. The light source 201 has a plurality of light-emitting modules evenly distributed on a same circumference. When there are two light sources 201, the light-emitting modules of the two light sources 201 are arranged alternately.

The imaging spectrum detection apparatus can image the imaging area 100 based on an instruction to obtain a corresponding image, and can obtain corresponding spectral data based on the instruction. The imaging spectrum detection apparatus includes a sensor and a periodic pixel-level light filtering structure arranged on a surface of the sensor. The periodic pixel-level light filtering structure is configured to perform spectral modulation on an incoming light signal, so that the sensor generates an image containing spectral information to be tested.

The periodic pixel-level light filtering structure includes a plurality of light filtering image element channels with pixel-level structures of different shapes. The plurality of light filtering image element channels have same specifications and sizes and are evenly arranged, and lengths and widths thereof are integer multiples of a pixel size in an image sensor. Light filtering image element channels of pixel-level light filtering structures of different shapes are corresponding to different spectral filter coefficients, and the pixel-level light filtering structures with different spectral filter coefficients are combined in a fixed order and then arranged periodically. The sensor modulates a received first testing light through the periodic pixel-level light filtering structure arranged on the surface of the sensor, to form a mosaic image containing spectral information, and then reconstructs a grayscale image including the spectral information to be tested using an algorithm.

The controller 203 is configured to control the light source 201 to provide light within a preset wavelength range to irradiate the first area, control the imaging spectrum detection apparatus to image the first area to obtain an image of an imaging area 100, and control the imaging spectrum detection apparatus to obtain, from the image, spectral data that indicate uneven distribution in the imaging area 100 of the reflection signal or excitation signal generated by the analyte when irradiated by light; and obtain information about the analyte in the imaging area 100 based on the obtained spectral data, where the information about the analyte includes information about the analyte correlated to the spectral data. When there is one light source 201, one image is formed. When there are two light sources 201, two images are formed. When the first light source 201 is turned on, the second light source 201 is turned off. Similarly, when the second light source 201 is turned on, the first light source 201 is turned off. The two light sources do not interfere with each other.

The first bandpass filter 204 is located between the light source 201 and the imaging area 100. A function of the first bandpass filter 204 is to allow light within a preset wavelength range to pass through, while cutting off light outside the preset wavelength range, thereby reducing the impact of other external light on a test result. In this implementation, the ring-shaped first bandpass filter 204 is preferred, and the ring shape of the first bandpass filter 204 is matched with the ring-shaped light source 201, so that light emitted by the light source 201 enters the imaging area 100 through the first bandpass filter 204.

The second bandpass filter 206 is located between the imaging spectrum detection apparatus 202 and the lens 205. A function of the second bandpass filter 206 is to allow light within a wavelength range in which the reflection signal or excitation signal generated by the analyte when irradiated by light is located to pass through, while cutting off light within another wavelength range, thereby reducing the influence of the reflection signal or excitation signal of a non-analyte on the test result.

The lens 205 can be configured to fix focus, to obtain a high-definition image. In another embodiment, the second bandpass filter 206 may alternatively be located on a side of the lens 205 away from the imaging spectrum detection apparatus 202, which is not limited in the present invention.

The illumination system in this embodiment can be integrated into the testing system and the testing device 200. The testing device 200 is a portable non-invasive testing device 200 for a human body, which may be a single testing device 200 or be integrated into a watch or mobile phone, to implement easy and rapid testing of the analyte in the surface of the human body.

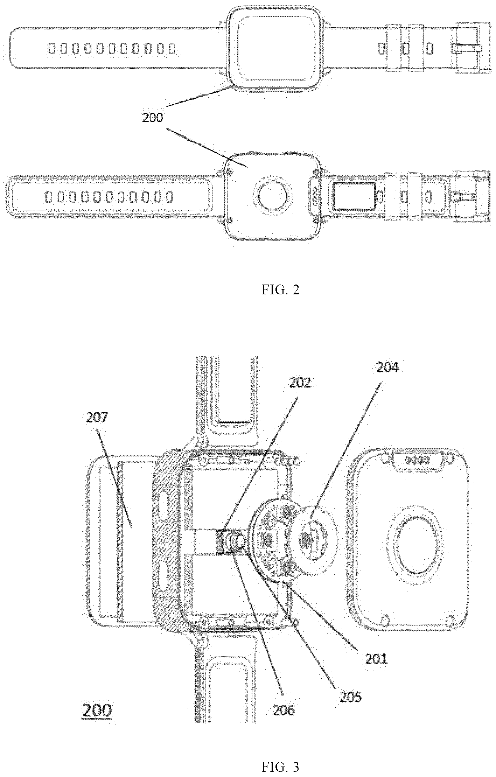

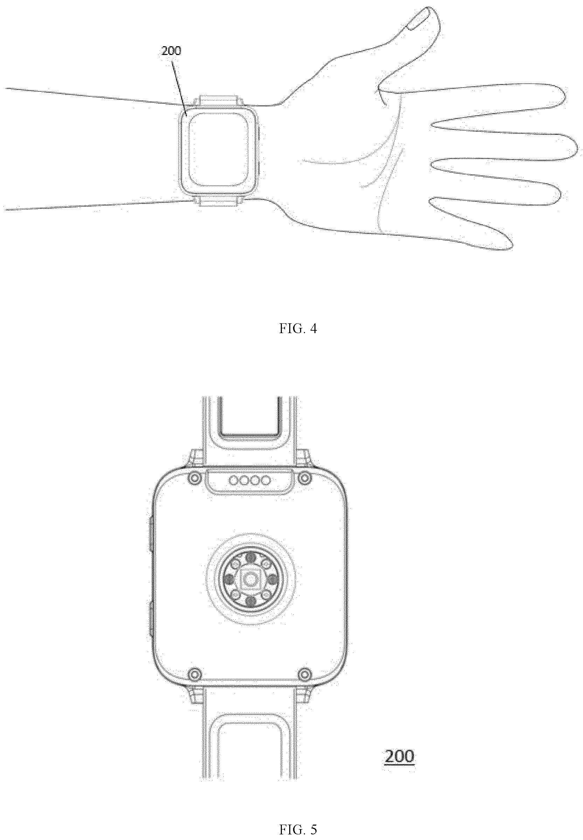

As shown in FIG. 2, FIG. 3, FIG. 4 and FIG. 5, for example, the testing device 200 in this embodiment is a watch, the system for testing is integrated in the watch, and the lens 205, the second bandpass filter 206, the light source 201, and the first bandpass filter 204 are arranged sequentially from the inside to the outside. The controller 203 is integrated on a circuit board 207 of the watch, and an opening area that fits the skin of a user's wrist is formed on a back housing of the watch. During testing, the imaging area 100 is a place where the opening area on the back of the watch fits the skin of the user's wrist. The reflection signal or excitation signal of the human body enters the light-transmitting window, passes through the light source 201 and a hollow part in the middle of the first bandpass filter 204, enters a second bandpass filter 206 through a lens 205, and enters an imaging spectrum detection apparatus 202 after being filtered by the second bandpass filter 206.

### Second embodiment

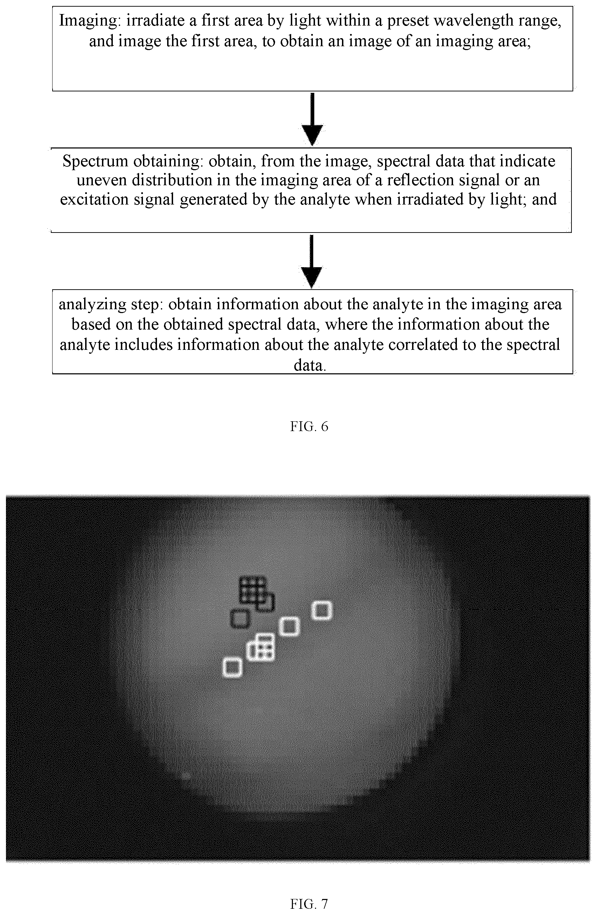

This embodiment provides a method for testing an analyte based on the first embodiment. FIG. 6 is a flow chart of this embodiment, and includes:
Imaging: providing light within a preset wavelength range through a light source to irradiate a first area, and imaging the first area through an imaging spectrum detection apparatus, to obtain an image of an imaging area. The image can demonstrate, during light irradiation within a preset wavelength range, distribution data and spectral data in the imaging area of a reflection signal or an excitation signal generated by the analyte when irradiated by light. The first area may be an area on a surface of a human skin. To prevent the influence of external light such as ambient light on testing, a lens of the imaging spectrum detection apparatus needs to be tightly attached to the surface of the human skin in the first area, and the imaging area means an area within a lens range of the imaging spectrum detection apparatus. In general, the imaging area may be a part of the first area, or may be the same area as the first area.

Because obtaining the distribution data and spectral data of the analyte requires irradiation by light of different wavelength ranges, there are two implementation manners: the light is light with a larger wavelength range provided by one light source or light with smaller wavelength ranges provided by two light sources respectively. When there is one light source, the wavelength range of the light provided by the light source needs to cover both a wavelength range within which analyte distribution data can be obtained and a wavelength range within which analyte spectral data can be obtained. When there are two light sources, the two light sources provide different pieces of light. A wavelength range of the analyte distribution data can be obtained through a wavelength coverage of one piece of light, and a wavelength range of the analyte spectral data can be obtained through a wavelength coverage of the other piece of light. In addition, when there is one light source, one image is formed; when there are two light sources, two images are formed. For ease of processing, imaging areas of the two images are required to be the same, that is, the lens of the imaging spectrum detection apparatus does not move on the surface of the human skin.

In this application, the analyte may be glucose, ketones, alcohol, lactate, oxygen, hemoglobin A1C, acetylcholine, amylase, bilirubin, cholesterol, chorionic gonadotropin, creatine kinase (such as CK-MB), creatine, creatinine, DNA, fructosamine, glutamine, growth hormone, hormone, peroxide, prostate-specific antigen, prothrombin, RNA, thyroid-stimulating hormone, or troponin in the blood vessel, or may be a drug such as antibiotics (such as gentamicin, vancomycin, or the like), digitoxin, digoxin, drugs of abuse, theophylline, or warfarin. In an implementation in which two or more analytes are tested, the analytes may be tested at the same or different time. In another embodiment, the analyte may alternatively be another substance on the surface of the human body, and non-invasive testing can be implemented according to the present invention.

Spectrum obtaining: obtaining, from the image by the imaging spectrum detection apparatus, spectral data that indicate uneven distribution in the imaging area of the reflection signal or the excitation signal generated by the analyte when irradiated by light. Specifically, the imaging area can be partitioned based on different pieces of distribution data, so that a position of the spectral data can be selected from different partitions.
analyzing step: obtaining information about the analyte in the imaging area based on the obtained spectral data, where the information about the analyte includes information about the analyte correlated to the spectral data. Due to a difference in distribution of the analyte in different areas, there is a difference in the reflection signal or excitation signal produced by the analytes when irradiated by light. For example, the human skin is divided into three parts: an epidermis tissue, a dermis tissue, and a subcutaneous tissue, and blood vessels such as veins are located in the subcutaneous tissue. Corresponding spectral data can be obtained by irradiating a skin area with blood vessels and a skin area without blood vessels irradiated by ultraviolet light, or the corresponding spectral data can be obtained from a skin area with thicker blood vessels and a skin area with thinner blood vessels. Therefore, a difference between the two pieces of spectral data can demonstrate the information about the analyte correlated to the spectral data in the blood vessels, such as intermediate information such as a degree of influence of the analyte on the spectral data for further analysis, or a concentration of the analyte and other information can be obtained directly through the analysis model.

### Third embodiment

This embodiment is based on the second embodiment. For example, glucose testing in human blood vessels is used as an example, and a non-invasive glucose testing method is provided, including the following steps.

Imaging: irradiating, by infrared light, skin on the wrist and the back of the hand at which the veins are located, in a first wavelength range of 800-1,000 nm, and collecting the first image of the imaging area, where the first wavelength range is preferably a near-infrared band; and irradiating, by ultraviolet light, a same position in a second wavelength range of 300-390 nm, to obtain the second image of the imaging area.

As shown in FIG. 7, an abscissa is a transverse coordinate of the first image, an ordinate is a longitudinal coordinate of the first image, white boxes represent selected testing point pixel blocks on venous blood vessels, and black boxes represent reference point pixel blocks on surrounding skin. In the first image, some of the infrared light penetrates the human skin and some is absorbed by the human skin, and is also absorbed by the venous blood vessels in large quantities in an area at which the venous blood vessels are located, presenting a characteristic that a pixel grayscale value of the area at which the venous blood vessels are located is small, and a pixel grayscale value of the area at which non-venous blood vessels are located is large, thereby easily dividing the imaging area into the area at which the venous blood vessels are located and the area at which the non-venous blood vessels are located.

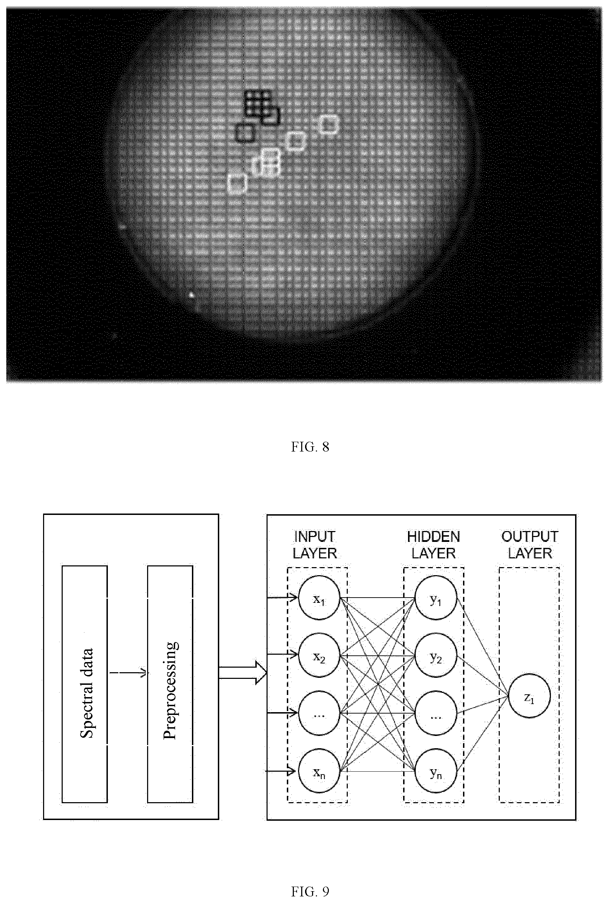

As shown in FIG. 8, an abscissa is a transverse coordinate of the second image, an ordinate is a longitudinal coordinate of the second image, white boxes represent selected testing point pixel blocks on the venous blood vessels, and black boxes represent reference point pixel blocks on surrounding skin. In the second image, because it is difficult to distinguish between the area at which the venous blood vessels are located and the area at which the non-venous blood vessels are located, the first image is required for distinguishing. Excitation light in the second wavelength range of 300-390 nm is used to obtain a high-quality effective fluorescence spectral signal. A main response band of the imaging spectrum detection apparatus is 400-800 nm. When the wavelength of the excitation light is less than 300 nm, a main peak of a fluorescence spectrum of the excited fluorescence radiation signal is in a band <400 nm, and it is difficult for the imaging spectrum detection apparatus to obtain the high-quality effective fluorescence spectral signal. When the wavelength of the excitation light used is >390 nm, the excitation light itself is visible light, the spectral signal of the excitation light and the fluorescence spectral signal are superimposed together, and it is difficult to eliminate the interference of the spectral signal of the excitation light and extract the effective fluorescence spectral signal. After absorbing ultraviolet light in the wavelength range of 300-390 nm, glucose in the venous blood vessels can emit a fluorescence radiation signal in a visible light band of 400-800 nm, and the band is in an effective response range of the imaging spectrum detection apparatus. A characteristic spectral intensity of the fluorescence radiation signal is positively correlated with the concentration of glucose, which has high fluorescence excitation efficiency.

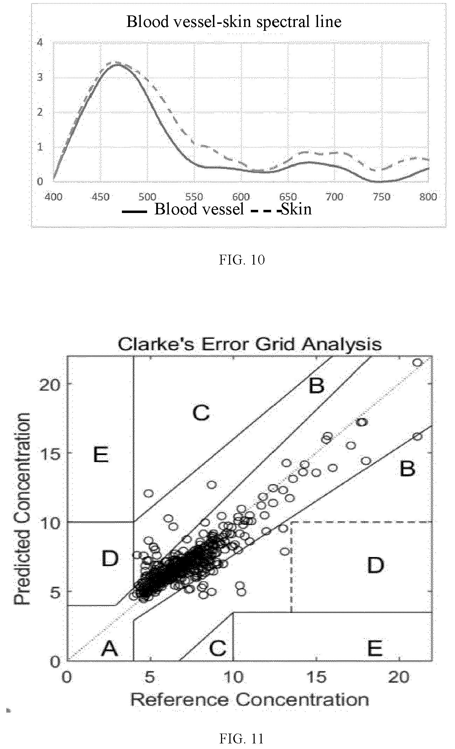

Spectral obtaining: based on grayscale distribution of pixels in the first image, dividing the imaging area into the area at which the venous blood vessels are located and the area at which the non-venous blood vessels are located, selecting a testing point from a position corresponding to the second image in the area at which the venous blood vessels are located, selecting a reference point from a position corresponding to the second image in the area at which the non-venous blood vessels are located, and respectively obtaining spectral data of the testing point and spectral data of the reference point in the second image. Specifically, based on a grayscale value of the pixel in the second image, a pixel whose grayscale value meets preset requirements is selected from the area at which the venous blood vessels are located as the testing point, or a combination of the pixel and an adjacent pixel is selected as the testing point, and a pixel whose grayscale value has a deviation from the selected testing point within a preset deviation range is selected from the area at which the non-venous blood vessels are located as the reference point, or a combination of the pixel and a plurality of adjacent pixels is selected as the reference point, and the fluorescence spectral data of the testing point and the fluorescence spectral data of the reference point are calculated in the second image. The spectral data is taken from the testing point, a pixel of the reference point, or a combined average of a plurality of pixels, which can be properly selected based on the width of the blood vessel. The combined average of the plurality of pixels can improve a signal-to-noise ratio, but is limited by the width of the blood vessel, preventing the spectral data from being taken from an extravascular area. Selection of a single pixel leads to high spatial resolution, which is proper for a thin blood vessel, but has a low signal-to-noise ratio. A preset requirement for the grayscale value may be using a point with the smallest grayscale value as the testing point, which is not limited in this application. A calculation result is shown in FIG. 10, an abscissa is the wavelength (in nm), an ordinate is a relative radiance (in W/nm), a solid line is the spectral data of the testing point, and a dashed line is the spectral data of the reference point. The grayscale value of the reference point and the grayscale value of the selected testing point are within the preset deviation range because the skin in the imaging area may be influenced by skin colors, pigmentation spots, cosmetics, and the like, which has a direct impact on the spectral data of the reference point, and the first image cannot distinguish the area including these influencing factors. These influencing factors can be effectively excluded by setting the preset deviation range of the grayscale value. In addition, the grayscale value and the grayscale value of the selected testing point are within the preset deviation range, which can ensure that the reference point is selected to be close to the testing point, such as an edge of the venous blood vessel. In this way, in addition to the blood vessels, the color, thickness, and other parameters of the epidermis tissue, the dermis tissue, and the subcutaneous tissues are approximately the same, so that a deviation between the spectral data of the testing point and the spectral data of the reference point can be excluded from the influence of a non-analyte as much as possible.

In addition to a spectrum reconstruction algorithm, the spectral data may alternatively be obtained by forming a radiometric calibration coefficient through previous radiometric calibration, and spectral lines are obtained by calculating the grayscale value * radiometric calibration coefficient.

When a combination of a plurality of pixels is selected at the testing point, the fluorescence spectral data at the testing point may be an average of fluorescence spectral data of these pixels. In addition, there may be one or more testing points and reference points. When there is a plurality of testing points and reference points, the average of the fluorescence spectral data of all testing points and the average of the fluorescence spectral data of all reference points can be calculated respectively.
analyzing step: preprocessing the obtained spectral data of the testing points and reference points, inputting the preprocessed data into a trained testing model, and outputting a concentration of glucose or an intermediate result of glucose correlated with the spectral data. When the testing model is trained, it is necessary to obtain spectral data and an accurate test result of a tested object, such as a blood collection test result, the spectral data is used as the input of the testing model, a blood collection test result is used as the output of the testing model, to train the testing model.

The testing model may be a convolutional neural network model, including an input layer, at least two convolutional layers, at least two activation function layers, a Flatten layer, a fully connected layer, and an output layer in sequence, and the convolutional layer and the activation function layer are spaced apart. An activation function used for the activation function layer is a Relu function.

In the convolutional neural network model, a size of each layer of convolutional kernels is 1, the number of convolutional kernels of a first convolutional layer is 32, and the number of convolutional kernels of a second layer is 64, which are both used to extract blood glucose features, and the output of the convolutional layer is transformed nonlinearly by the activation function. The Flatten layer flattens the output of the convolutional layer into a one-dimensional vector, to connect the subsequent fully connected layers, resulting in a final output dimension of 1. The Adam optimizer is used for model training during model training, a mean square error is used as a loss function, and a mean absolute error is calculated as a performance indicator of model evaluation.

When an output result of the testing model is glucose concentration, if an error between the output result and a measured standard glucose concentration value meets a preset condition, training is stopped, to obtain the testing model. When the output result of the testing model is the intermediate result of glucose correlated to the spectral data, such as an intermediate neuron result, if the error between the output result and the intermediate neuron result meets the preset condition, training is stopped to obtain the testing model. The intermediate neuron result is further model-corrected to obtain the concentration of glucose.

As shown in FIG. 9, the input layer is a spectral data input layer, which is obtained after the original spectral data is preprocessed. A hidden layer is a middle hidden layer, and a finally predicted blood glucose concentration value is output in an output layer after feature combination through convolution operation deep learning. Alternatively, through convolution operation deep learning, a neuron Output1 is output as an intermediate result value after feature combination, and model training is performed again on the intermediate result values Output1 and two infrared IR feature brightness values, to further correct a blood glucose prediction error, and the finally predicted blood glucose concentration value Output2 is output. Different parameters need to be set for a training degree of the testing model according to the needs, and a plurality of extracted glucose eigenvalues are continuously learned based on setting of different parameters, until an error between an output result and a standard glucose value of the above label value meets the requirements, and then training is stopped, to obtain the testing model.

Through a plurality of iterations of training, neurons can learn corresponding changes between different glucose concentrations and glucose spectral characteristics of different sampled objects, thereby improving the universality of the testing model and implementing prediction of glucose concentrations for different users.

In the whole glucose testing process, there is no need to pierce the skin for collecting blood or pierce the skin for implantation, and spectral information of a person to be tested is obtained based on the fluorescence spectrum, and a glucose test result of the person to be tested is obtained based on the spectral information, preventing pain and discomfort, and improving testing comfort and convenience. In the method, a spectral signal for blood vessels and a spectral signal for the skin can be finely distinguished, which provides a possibility for subsequent accurate extraction of a glucose signal, and also enables the spectral signal to be strongly correlated with the glucose concentration, implementing accurate measurement of the glucose concentration, so that a test result is more accurate, and processing is more easily performed.

FIG. 11 shows a schematic diagram of an experimental effect of the trained testing model, where an abscissa is a reference blood glucose concentration (in mmol/L) collected by a blood glucose meter, and an ordinate is a blood glucose concentration (in mmol/L) predicted in the method in the patent. There are totally 2,037 samples of tested objects, including 1,537 samples from a training set and 500 samples from a prediction set. Distribution of test results for the testing model can be learned from the figure, a MARD value of the predicted samples is 11.32%, and most of the samples fall in areas A and B, among which 87.03% of the samples fall in area A and 12.77% of the samples fall in area B, indicating that testing accuracy of the testing model is high.

### Fourth embodiment

This embodiment is based on the third embodiment. Infrared light is replaced with visible light, and another non-invasive method for glucose testing is provided, including the following steps.

Imaging: collecting the first image of the imaging area by irradiating visible light on the skin, such as the wrist and the back of the hand, at which the veins are located; and irradiating, by ultraviolet light, a same position in a second wavelength range of 300-390 nm, to obtain the second image of the imaging area.

In the first image, due to the difference between a color of the area at which the venous blood vessels are located and a color of the area at which the non-venous blood vessels are located, the imaging area can be easily divided into the area at which the venous blood vessels are located and the area at which the non-venous blood vessels are located.

In the second image, because it is difficult to distinguish between the area at which the venous blood vessels are located and the area at which the non-venous blood vessels are located, the first image is required for distinguishing. Excitation light in the second wavelength range of 300-390 nm is used to obtain a high-quality effective fluorescence spectral signal. A main response band of the imaging spectrum detection apparatus is 400-800 nm. When the wavelength of the excitation light is less than 300 nm, a main peak of a fluorescence spectrum of the excited fluorescence radiation signal is in a band <400 nm, and it is difficult for the imaging spectrum detection apparatus to obtain the high-quality effective fluorescence spectral signal. When the wavelength of the excitation light used is >390 nm, the excitation light itself is visible light, the spectral signal of the excitation light and the fluorescence spectral signal are superimposed together, and it is difficult to eliminate the interference of the spectral signal of the excitation light and extract the effective fluorescence spectral signal. After absorbing ultraviolet light in the wavelength range of 300-390 nm, glucose in the venous blood vessels can emit a fluorescence radiation signal in a visible light band of 400-800 nm, and the band is in an effective response range of the imaging spectrum detection apparatus. A characteristic spectral intensity of the fluorescence radiation signal is positively correlated with the concentration of glucose, which has high fluorescence excitation efficiency.

Spectral obtaining: based on grayscale distribution of pixels in the first image, dividing the imaging area into an area at which the venous blood vessels are located and an area at which the non-venous blood vessels are located, selecting a testing point from a corresponding second image in the area at which the venous blood vessels are located, selecting a reference point from a corresponding second image in the area at which the non-venous blood vessels are located, and respectively obtaining spectral data of the testing point and spectral data of the reference point. Specifically, based on a grayscale value of the pixel in the second image, a pixel whose grayscale value meets preset requirements is selected from the area at which the venous blood vessels are located as the testing point, or a combination of the pixel and an adjacent pixel is selected as the testing point, and a pixel whose grayscale value has a deviation from the selected testing point within a preset deviation range is selected from the area at which the non-venous blood vessels are located as the reference point, or a combination of the pixel and a plurality of adjacent pixels is selected as the reference point, and the fluorescence spectral data of the testing point and the fluorescence spectral data of the reference point are calculated. The grayscale value of the reference point and the grayscale value of the selected testing point are within the preset deviation range because the skin in the imaging area may be influenced by skin colors, pigmentation spots, cosmetics, and the like, which has a direct impact on the spectral data of the reference point, and the first image cannot distinguish the area including all influencing factors. These influencing factors can be effectively excluded by setting the preset deviation range of the grayscale value.

When a combination of a plurality of pixels is selected at the testing point, the fluorescence spectral data at the testing point may be an average of fluorescence spectral data of these pixels. In addition, there may be one or more testing points and reference points. When there is a plurality of testing points and reference points, the average of the fluorescence spectral data of all testing points and the average of the fluorescence spectral data of all reference points can be calculated respectively.
analyzing step: preprocessing the obtained spectral data of the testing points and reference points, inputting the preprocessed data into a trained testing model, and outputting the concentration of glucose. When the testing model is trained, it is necessary to obtain spectral data and an accurate test result of a tested object, such as a blood collection test result, the spectral data is used as the input of the testing model, a blood collection test result is used as the output of the testing model, to train the testing model.

The testing model may be a convolutional neural network model, including an input layer, at least two convolutional layers, at least two activation function layers, a Flatten layer, a fully connected layer, and an output layer in sequence, and the convolutional layer and the activation function layer are spaced apart. An activation function used for the activation function layer is a Relu function.

In the convolutional neural network model, a size of each layer of convolutional kernels is 1, the number of convolutional kernels of a first convolutional layer is 32, and the number of convolutional kernels of a second layer is 64, which are both used to extract blood glucose features, and the output of the convolutional layer is transformed nonlinearly by the activation function. The Flatten layer flattens the output of the convolutional layer into a one-dimensional vector, to connect the subsequent fully connected layers, resulting in a final output dimension of 1. The Adam optimizer is used for model training during model training, a mean square error is used as a loss function, and a mean absolute error is calculated as a performance indicator of model evaluation.

If an error between the output result of the testing model and a standard glucose concentration meets a preset condition, training is stopped, to obtain the testing model.

Different parameters need to be set for a training degree of the testing model according to the needs, and a plurality of extracted glucose eigenvalues are continuously learned based on setting of different parameters, until an error between an output result and a standard glucose value of the above label value meets the requirements, and then training is stopped, to obtain the testing model.

Through a plurality of iterations of training, neurons can learn corresponding changes between different glucose concentrations and glucose spectral characteristics of different sampled objects, thereby improving the universality of the testing model and implementing prediction of glucose concentrations for different users.

In the whole glucose testing process, there is no need to collect blood or pierce the skin, and spectral information of a person to be tested is obtained based on the fluorescence spectrum, and a glucose test result of the person to be tested is obtained based on the spectral information, preventing pain and discomfort, and improving testing comfort and convenience. In the method, a spectral signal for blood vessels and a spectral signal for the skin can be finely distinguished, which provides a possibility for subsequent accurate extraction of a glucose signal, and also enables the spectral signal to be strongly correlated with the glucose concentration, implementing accurate measurement of the glucose concentration, so that a test result is more accurate, and processing is more easily performed.

### Fifth embodiment

This embodiment provides a system for testing an analyte based on the first embodiment. The system for testing an analyte can be implemented by performing flow steps of the method for testing an analyte. In other words, those skilled in the art can understand the method for testing an analyte as a preferred implementation of the system for testing an analyte. The system for testing an analyte includes the following modules.

An imaging module, configured to provide light within a preset wavelength range through a light source to irradiate a first area, and image the first area through an imaging spectrum detection apparatus, to obtain an image of an imaging area. The image can demonstrate, during light irradiation within a preset wavelength range, distribution data and spectral data in the imaging area of a reflection signal or an excitation signal generated by the analyte when irradiated by light. Because different wavelength ranges are required to obtain distribution data and spectral data of the analyte, the light may be either light corresponding to two wavelength ranges or light with a larger wavelength range that covers the two desired wavelength ranges. When there are two pieces of light, there are two obtained images. To facilitate processing, it is usually required that imaging areas of the two images be the same.

In this application, the analyte may be glucose, ketones, alcohol, lactate, oxygen, hemoglobin A1C, acetylcholine, amylase, bilirubin, cholesterol, chorionic gonadotropin, creatine kinase (such as CK-MB), creatine, creatinine, DNA, fructosamine, glutamine, growth hormone, hormone, peroxide, prostate-specific antigen, prothrombin, RNA, thyroid-stimulating hormone, or troponin in the blood vessel of an animal, or may be a drug such as antibiotics (such as gentamicin, vancomycin, or the like), digitoxin, digoxin, drugs of abuse, theophylline, and warfarin. In an implementation in which one or more analytes are tested, the analytes may be tested at the same or different time. In other embodiments, the analyte may alternatively be another substance in liquid.

A spectral obtaining module, configured to obtain, from the image by the imaging spectrum detection apparatus, spectral data that indicate uneven distribution in the imaging area of the reflection signal or the excitation signal generated by the analyte when irradiated by light. Specifically, the imaging area can be partitioned based on different pieces of distribution data, so that a position of the spectral data can be selected from different partitions.

An analysis module, configured to obtain information about the analyte in the imaging area based on the obtained spectral data, where the information about the analyte includes information about the analyte correlated to the spectral data. Due to a difference in distribution of the analyte in different areas, there is a difference in the reflection signal or excitation signal produced by the analytes when irradiated by light. With this feature, the difference between the two pieces of spectral data can be obtained, to accurately demonstrate the information about the analyte correlated to the spectral data, such as the concentration of the analyte.

Those skilled in the art know that, in addition to implementing the system provided in the present invention and each apparatus, module, and unit thereof in a purely computer-readable program code mode, the system provided in the present invention and each apparatus, module, and unit thereof can be enabled to implement same functions in the form of logic gates, switches, application-specific integrated circuits, programmable logic controllers, and embedded microcontrollers by performing logic programming of the method steps. Therefore, the system provided in the present invention and each apparatus, module, and unit thereof may be regarded as hardware components, and each apparatus, module, and unit thereof for implementing various functions may also be regarded as structures within the hardware components; and each apparatus, module, and unit thereof for implementing various functions may alternatively be regarded as software modules for implementing methods or structures within the hardware components.

### Sixth embodiment

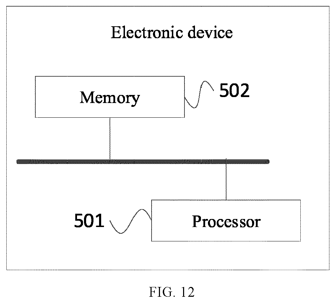

This embodiment provides a schematic structural diagram of an electronic device based on the first embodiment. As shown in FIG. 12, the electronic device includes at least one processor 501 and a memory 502 that is in communication connection with the at least one processor 501. The memory 502 stores instructions that can be executed by the at least one processor 501. The instructions are executed by the at least one processor 501, so that the at least one processor 501 can perform the above method for testing an analyte.

The memory 502 and the processor 501 are connected using a bus. The bus may include interconnected buses and bridges of any quantities. The bus connects various circuits of one or more processors 501 and memories 502. The bus may further connect a peripheral device, a voltage regulator, and various other circuits such as a power management circuit, which are all well known in the art and are not further described in the present invention. A bus interface provides an interface between the bus and a transceiver. The transceiver may be one component or a plurality of components, for example, a plurality of receivers and transmitters, to provide a unit that is configured to communicate with various other apparatuses on a transmission medium. Data processed by the processor 501 is transmitted on a wireless medium by using the antenna. Further, the antenna further receives data and transmits the data to the processor 501.

The processor 501 is responsible for managing the bus and general processing, and may further provide various functions, including timing, peripheral interfacing, voltage regulation, power management, and another control function. The memory 502 may be configured to store data used by the processor 501 when performing an operation.

The present invention also provides a computer-readable storage medium that stores a computer program, and when the computer program is executed by a processor, the above method for testing an analyte is implemented.

To be specific, those skilled in the art can understand that all or some of steps in the method of the above embodiments can be completed by instructing relevant hardware through a program. The program is stored in a storage medium and includes several instructions to enable a device (which may be a single-chip microcontroller, a chip, or the like) or a processor (processor) to perform all or some of the steps of the method described in various embodiments of this application. The above storage medium includes any medium that can store program code, such as a USB flash drive, a removable hard disk, a read-only memory (ROM, Read-Only Memory), a random access memory (RAM, Random Access Memory), a magnetic disk, or an optical disc.

Those skilled in the art can understand that the above implementations are specific embodiments for implementing the present invention, and in actual application, various changes may be made thereto in form and detail without departing from the spirit and scope of the present invention.

In the descriptions of this application, it should be understood that an orientation or a position relationship indicated by the terms "above", "below", "front", "back", "left", "right", "vertical", "horizontal", "top", "bottom", "inside", "outside", and the like is based on an orientation or a position relationship shown in the accompanying drawings, and is merely intended for ease of describing this application and simplifying description, but does not indicate or imply that a described apparatus or element needs to have a specific orientation or be constructed and operated in a specific orientation. Therefore, such terms shall not be understood as a limitation on this application.

Specific embodiments of the present invention are described above. It should be understood that the present invention is not limited to the foregoing specific implementations. Those skilled in the art can make various variations or modifications within the scope of the claims, which does not affect the essence of the present invention. Embodiments in this application and the features in embodiments may be arbitrarily and mutually combined in the case of no conflict.

## Claims

1. A system for illuminating an analyte, comprising a shell and a light source (201), wherein the light source (201) is capable of providing light within a preset wavelength range; and
the shell is capable of fitting a tested part of a tested object and forming an imaging area (100) at a fitting position, and the light source (201) illuminates the imaging area (100).

2. The system for illuminating an analyte according to claim 1, wherein the light source (201) comprises a single light source (201), and a wavelength range of light provided by the single light source (201) covers both a wavelength range within which analyte distribution data can be obtained and a wavelength range within which analyte spectral data can be obtained.

3. The system for illuminating an analyte according to claim 2, wherein the light source (201) comprises a plurality of light-emitting modules, which are evenly distributed on a same circumference.

4. The system for illuminating an analyte according to claim 1, wherein the light source (201) comprises two light sources (201), a wavelength range of light provided by one light source (201) covers a wavelength range within which analyte distribution data can be obtained, and a wavelength range of light provided by the other light source (201) covers a wavelength range within which analyte spectral data can be obtained.

5. The system for illuminating an analyte according to claim 4, wherein the two light sources (201) respectively comprise a plurality of light-emitting modules, the plurality of light-emitting modules of the two light sources (201) are arranged alternately on a same circumference, and the light-emitting modules are evenly distributed on the same circumference.

6. The system for illuminating an analyte according to claim 4, wherein one light source (201) provides infrared light with a wavelength range from 800 nm to 1,000 nm; and
the other light source (201) provides ultraviolet light with a wavelength range of 300 nm to 390 nm.

7. The system for illuminating an analyte according to claim 1, wherein a first bandpass filter (204) is also disposed in the shell, and the first bandpass filter (204) is located between the light source (201) and the imaging area (100); and
the first bandpass filter (204) allows the light within the preset wavelength range to pass through and light outside the preset wavelength range to be cut off.

8. The system for illuminating an analyte according to claim 1, wherein the shape and material of the shell allow the shell to fit the skin around a perimeter of the tested part of the tested object.

9. A system for testing an analyte, comprising the system for illuminating an analyte according to any one of claims 1 to 8.

10. A system for testing an analyte, further comprising a portable apparatus, wherein a system for illuminating an analyte is integrated in the portable apparatus.
